# DEMANDE DE BREVET EUROPEEN

(11) **EP 2 910 277 A1**
(43) Date de publication de la demande: **26.08.2015**
(21) Numéro de dépôt: 15155870.7
(22) Date de dépôt: 20.02.2015
(51) Int. Cl.: A61N 1/32

(54) **APPAREIL ÉLECTRIQUE FORMANT GÉNÉRATEUR D'IMPULSIONS ÉQUIPÉ D'ÉLECTRODES DE TRAITEMENT ESTHÉTIQUE DE LA PEAU**

(30) Priorité: 21.02.2014 CH 2422014
(71) Demandeur: P.S. Cosmetique Instrumentale SA, 1206 Geneve (CH)
(72) Inventeur: Simonin, Philippe, 75017 Paris (FR)
(74) Mandataire: Cronin, Brian Harold John

(57) **Abrégé**

Un appareil de stimulation et de traitement de la peau, comporte des moyens générateurs (11) pour élaborer un signal haute fréquence ayant une fréquence de l'ordre de grandeur de 1 MHz, soit de 0,7MHz à 1,3MHz, modulé en amplitude par un signal basse fréquence ayant une fréquence de l'ordre de grandeur de 50 Hz, soit de 45Hz à 65Hz, sous une tension variable de 200V à 1200V. Une première électrode (23), montée sur un stylo porte-électrode de forme cylindrique, est reliée à la sortie desdits moyens générateurs (11) et est apte à être déplacée sur une première zone de peau à traiter tout en lui appliquant le signal. Une seconde électrode (24), montée sur un stylo masse porte-électrode de forme cylindrique, est reliée à la masse et est apte à être déplacée sur une zone de peau à traiter adjacente à ladite première zone. La première électrode (23) est apte à appliquer le signal haute fréquence à la seconde électrode (24) à travers la peau. Les deux électrodes (23, 24) comportent chacune une zone d'application allongée continue ou discontinue pour venir pincer la peau. Les deux électrodes (23, 24) sont maniables par un utilisateur pour venir se placer de part et d'autre d'une zone de la peau à traiter afin de pincer la peau dans cette zone pour le traitement.

## Description

L'invention concerne un appareil électrique formant générateur d'impulsions équipé d'électrodes de traitement esthétique de la peau, notamment des rides, permettant d'appliquer lesdites impulsions, au moyen d'électrodes de forme adaptée, à la surface de la peau, pour des traitements localisés, notamment des rides, cicatrices, dépression de la peau et cernes.

On connaît déjà divers procédés et moyens de traitement esthétique ou de rajeunissement de la peau qui permettent l'effacement au moins temporaire des rides, plissements ou analogues apparaissant sur le corps humain, et notamment sur le visage, à partir d'un certain âge.

C'est ainsi qu'il est déjà connu de rajeunir la peau du visage par application topique de certains produits cosmétiques ou bien par meulage ou emploi de moyens abrasifs divers permettant d'effacer les rides ou irrégularités diverses apparaissant sur l'épiderme.

Par ailleurs, il est connu de provoquer une électrostimulation de la peau au moyen d'aiguilles alimentées par un courant de faible intensité ou encore au moyen d'électrodes en forme de disques qui sont appliquées sur la peau pour procurer une faible vibration électrique agissant sur les muscles et tendant à atténuer les rides ou les plissements de la peau.

Mais, dans le premier cas, il y a piqûre et donc risque de saignement, et, dans le deuxième cas, les électrodes en forme de disques souples reliés au courant galvanique ou de basse fréquence excitomoteur ne peuvent pas prétendre exercer une action rigoureusement précise et bien déterminée sur le défaut ou l'irrégularité de la peau à traiter.

On connaît du brevet français FR2753385 un appareil électrique formant générateur d'impulsions pour, notamment, le traitement des rides.

Le document EP0592851 décrit un dispositif de stimulation et traitement ayant une électrode connectée aux moyens générateurs ayant une fréquence de l'ordre de grandeur de 1 MHz, modulée en amplitude par un signal basse fréquence ayant une fréquence de l'ordre de grandeur de 50 Hz.

EP1385572 décrit un appareil de stimulation et de traitement de la peau, caractérisé en ce qu'il comporte des moyens générateurs pour élaborer un signal haute fréquence modulé en amplitude par un signal basse fréquence. Pour le traitement des rides avec cet appareil, le praticien doit appliquer sur la partie à traiter du sujet une électrode en forme de T dite luge, pendant que le sujet tient fermement en main une électrode de masse. Cette méthode nécessite donc l'intervention du sujet.

D'autre part, US20120290044A1 décrit une méthode pour la tonification des muscles faciaux utilisant un courant galvanique à une fréquence de 3 à 10 Hz appliquée à la peau par des électrodes dont les extrémités sont mouillées par une solution conductrice pour conduire le courant galvanique à basse fréquence.

Par contre, la présente invention a pour but de proposer un appareil de stimulation et de traitement de la peau, sans tonification musculaire, avec des résultats très significatifs, notamment par un traitement des rides par pincement des rides entre deux électrodes appliquées directement sur la peau sans liquide conducteur.

Plus précisément, l'invention concerne un appareil de stimulation et de traitement de la peau, comportant des moyens générateurs pour élaborer un signal haute fréquence ayant une fréquence de l'ordre de grandeur de 1 MHz, soit de 0,7MHz à 1,3MHz, modulé en amplitude par un signal basse fréquence ayant une fréquence de l'ordre de grandeur de 50 Hz, soit de 45Hz à 65Hz, sous une tension variable de 200V à 1200V. Une première électrode, montée sur un stylo porte-électrode de forme cylindrique, est reliée à la sortie desdits moyens générateurs et est apte à être déplacée sur une première zone de peau à traiter tout en lui appliquant le signal. Une seconde électrode, montée sur un stylo masse porte-électrode de forme cylindrique, est reliée à la masse et est apte à être déplacée sur une zone de peau à traiter adjacente à ladite première zone. La première électrode est apte à appliquer le signal haute fréquence à la seconde électrode à travers la peau. Les deux électrodes comportent chacune une zone d'application allongée continue ou discontinue pour venir pincer la peau. Les deux électrodes sont maniables par un utilisateur pour venir se placer de part et d'autre d'une zone de la peau à traiter afin de pincer la peau dans cette zone pour le traitement.

L'utilisateur prend en main les deux électrodes qu'il vient placer de part et d'autre d'une zone de la peau à traiter. Ainsi, la peau est pincée entre les deux électrodes et le courant passe entre les deux électrodes ce qui a pour effet d'être extrêmement efficace pour le traitement des rides, cicatrices, dépressions de la peau et cernes, sans besoin d'une tonification musculaire, puisque le champ électrique agit dans la zone pincée de la peau.

Selon un mode de réalisation possible, lesdites électrodes d'application ont une forme triangulaire. Selon une autre variante possible, les électrodes ont la forme d'un T ou d'un L, soit des électrodes dont la branche est terminée par une barrette s'étendant transversalement.

Le signal haute fréquence a une fréquence de l'ordre de grandeur de 1 MHz, soit de 0.7MHz à 1.3MHz, tandis que le signal basse fréquence a un ordre de grandeur de 50 Hz, soit de 45Hz à 65Hz. Avantageusement, le signal basse fréquence peut être obtenu à partir de la tension alternative du réseau de distribution de l'électricité.

Pour combiner les deux signaux de fréquences différentes, on peut prévoir un hacheur piloté à ladite haute fréquence et recevant en entrée ledit signal basse fréquence. Le signal résultant peut être appliqué à la première électrode via un transformateur HF, dit transformateur d'impulsions, connecté entre le hacheur et ladite première électrode. Ce transformateur d'impulsions, de faible puissance, permet d'élever la tension appliquée à la première électrode tout en limitant la valeur du courant à une valeur faible non dangereuse.

Lorsque le traitement est opéré par un praticien, l'appareil est muni d'un stylo masse porte-électrode sur lequel est montée la seconde électrode, reliée par un fil électrique à l'une des bornes du générateur d'impulsions. De préférence, la seconde électrode employée est de forme triangulaire. L'autre borne du générateur est reliée par un autre fil électrique à un stylo porte-électrode portant une première électrode. De préférence, la première électrode sera de la même forme que la seconde électrode, en l'occurrence de forme triangulaire de préférence. Le praticien prend les deux électrodes en main et les mets en contact avec la peau du sujet en les plaçant de part et d'autre d'une zone de la peau à traiter afin de pincer la peau dans cette zone pour le traitement. Cette opération du pincement de la peau se fait directement sans utilisation de solution conductrice. Pendant le traitement par pincement des rides entre la première et la seconde électrodes, le sujet n'a pas besoin d'intervenir. En effet, les deux électrodes sont maniables par un utilisateur pour venir se placer de part et d'autre d'une zone de la peau à traiter afin de pincer la peau dans cette zone pour le traitement, permettant ainsi de localiser le traitement et de le rendre plus efficace.

Par contre, pour une autre application accessoire autre que le traitement cutané par pincement entre deux électrodes, la seconde électrode est démontée et remplacée par une masse cylindrique qui s'emboite dans le stylo masse porte-électrode. Dès lors, le sujet tient le stylo masse porte-électrode à la main pendant que le praticien déplace la première électrode sur la zone de la peau à traiter.

Les électrodes sont donc amovibles et on peut disposer d'un jeu d'électrodes de formes différentes, chaque électrode étant adaptable audit embout de raccordement. La forme de l'électrode est choisie en fonction de la nature du traitement et des résultats escomptés.

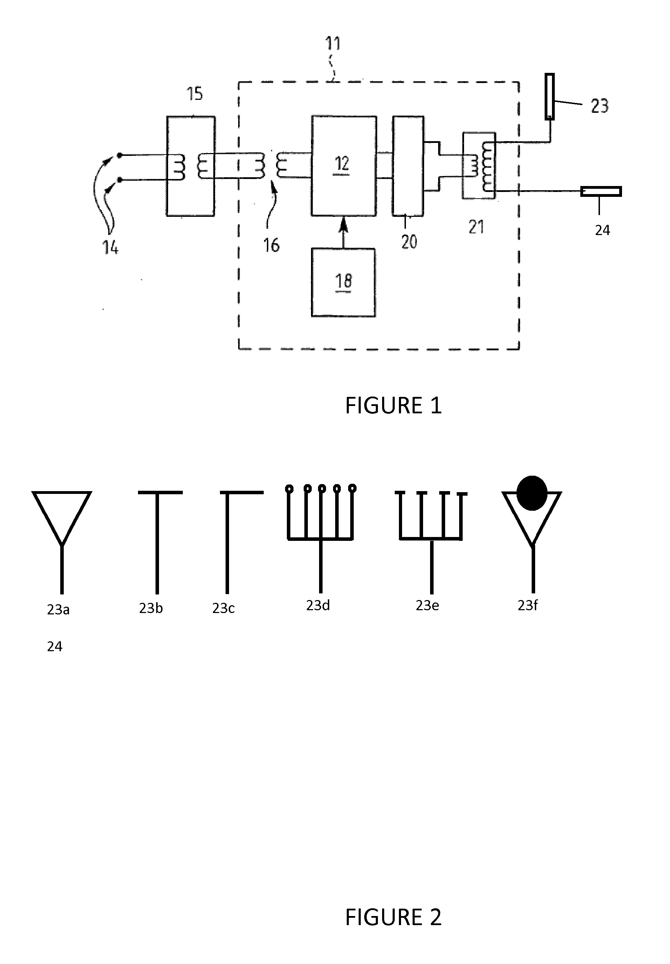

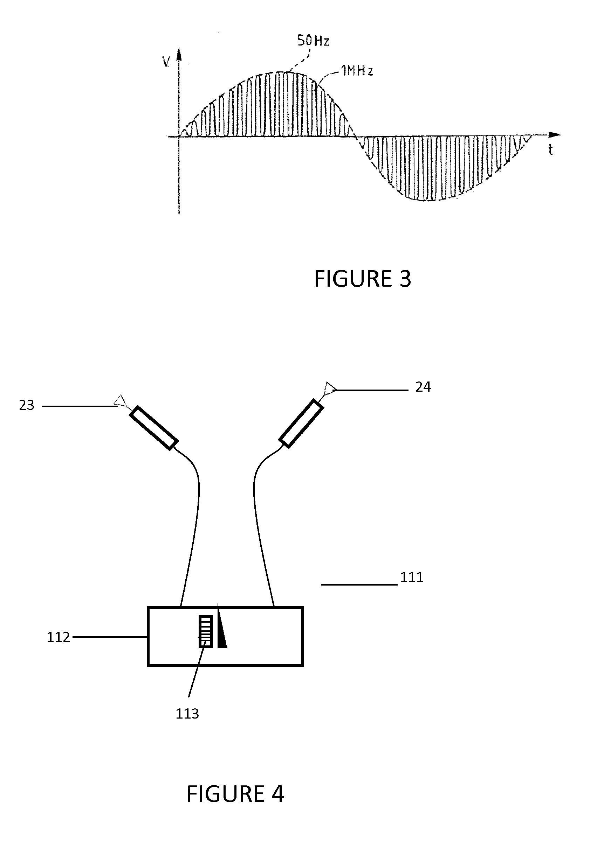

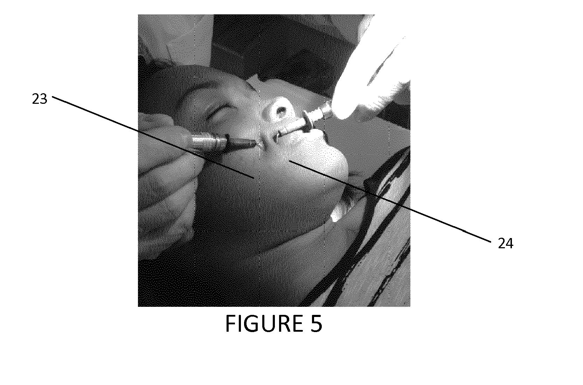

L'invention sera mieux comprise et d'autres avantages de celle-ci apparaîtront plus clairement à la lumière de la description qui va suivre d'un mode de réalisation possible d'un appareil conforme à son principe, donnée uniquement à titre d'exemple, et faite en référence aux dessins annexés dans lesquels :
l a figure 1 est un schéma bloc du générateur permettant d'élaborer le signal électrique désiré ;
l a figure 2 est un exemple d'électrodes applicables ;
l a figure 3 est un graphe illustrant la variation du signal délivré par le générateur de la figure 1 ;
l a figure 4 illustre un équipement complet pour la mise en oeuvre d'un traitement pour le traitement des rides par pincement ; et
l a figure 5 illustre un sujet traité pour les rides par le pincement desdites rides entre deux électrodes.

Sur la figure 1, on a représenté un générateur 11 comportant un hacheur 12 recevant en entrée une tension alternative à la fréquence de 50 Hz. Cette tension alternative peut être prélevée sur le réseau de distribution de courant, par exemple par l'intermédiaire d'un transformateur 15, abaisseur de tension situé à l'extérieur du boîtier du générateur 11. Le générateur 11 est relié au réseau de distribution de courant alternatif 14 (220V, 50Hz) par le transformateur abaisseur de tension 15 délivrant une basse tension de 12V, 50 Hz. Le transformateur 15 alimente un transformateur élévateur 16, situé à l'intérieur du boîtier du générateur, qui restitue une tension de 220V environ, à l'entrée du hacheur 12. Par ailleurs, un oscillateur 18 de 1 MHz est connecté à l'entrée de pilotage du hacheur. Il en résulte que le signal de sortie du hacheur est un signal haute fréquence (1 MHz) modulé en amplitude par le signal basse fréquence (50 Hz). Ce signal de sortie est appliqué à un circuit régulateur 20, limiteur de courant, dont la sortie est connectée à l'enroulement primaire d'un transformateur haute fréquence 21 : dit transformateur d'impulsions, élévateur de tension. L'une des bornes de l'enroulement secondaire est connectée à une électrode 23 montée sur un stylo porte-électrode, métallique, tandis que l'autre borne est connectée à un stylo masse porte-électrode sur laquelle est montée une électrode de forme triangulaire 24 ou une masse métallique destinée à être mise en contact avec l'épiderme de la personne en cours de traitement.

Pour le traitement des rides par pincement, le praticien place l'électrode 23, de préférence de forme triangulaire, et la seconde électrode 24 de préférence triangulaire de part et d'autre d'une ride pour la pincer, telle qu'un individu ferait pour se percer un bouton qu'il aurait sur le corps (figure 5). Cette opération du pincement de la peau se fait directement sans utilisation de solution conductrice. Le signal V disponible à la sortie du hacheur est du type illustré à la figure 3. Les oscillations de haute fréquence ici à 1MHz évoluent en amplitude à l'intérieur d'une enveloppe de basse fréquence (50Hz). Bien entendu, les rapports des fréquences ne sont pas respectés sur la figure 3. Les alternances à 1 MHz s'inscrivent en amplitude dans une enveloppe à 50 Hz.

On peut penser que le signal haute fréquence, une fois élevé en tension et appliqué sur la peau, procure une stimulation des couches basales de l'épiderme tandis que la modulation de cette haute fréquence (l'enveloppe basse fréquence) "traverse" la barrière épidermique, en stimulant les fibroblastes ce qui favorise la production de collagène et d'élastine. La haute fréquence, quant à elle, favorise la microcirculation artérielle veineuse et lymphatique. Si les résultats observés avec EP1385572 sont très bons, les résultats observés avec cette nouvelle technique de pincement de la ride entre deux électrodes, notamment grâce à une électrode de masse modifiée, sont nettement meilleurs. En effet, l'intensité fournie par l'appareil électrique formant générateur est concentrée dans une zone localisée de la peau, entre les électrodes ce qui a pour effet d'accroitre l'efficacité du traitement.

Cette stimulation par haute fréquence selon l'invention se fait sans le passage d'un courant galvanique mais, sans être lié à une théorie, on peut assimiler l'action à un mécanisme d'induction.

Ce type de traitement, c'est à dire le fait d'appliquer ce signal sous une tension variable de 200 à 1200 V, via le transformateur d'impulsions, notamment par le pincement des rides entre deux électrodes, donne des résultats très significatifs pour la diminution des rides superficielles, moyennes et profondes tout en améliorant la tonicité cutanée.

Accessoirement, lorsque l'appareil est employé avec une seule électrode et le sujet traité tient une masse à la main, on obtient de bons résultats sur le métabolisme des cheveux, notamment en ayant un effet sur le ralentissement de la chute, la régulation de la fonction sébacée et sudoripare, l'augmentation du volume des cheveux, amélioration de la qualité des cheveux, repousse plus rapide des cheveux, notamment dans le cas d'implants.

La figure 2 montre différents types d'électrodes possibles utilisables chacune pour un traitement particulier. Ainsi, les électrodes 23a, 23b, 23c seraient les mieux adaptées pour le traitement des rides par pincement. De préférence, l'électrode 23 pour traiter les rides sera de forme triangulaire et la seconde électrode 24 sera également de forme triangulaire. Les électrodes 23d, 23e, 23f sont quant à elles utilisées pour des traitements capillaires lorsque la seconde électrode 24 est remplacée par une masse tenue en main par le sujet. Par exemple, l'électrode 23d de la figure 2 a la forme générale d'une fourche comprenant plusieurs branches 27 parallèles (5 dans l'exemple représenté) et chaque branche est terminée par une petite excroissance 28, par exemple sensiblement sphérique. L'électrode 23e de la figure 2 a également la forme générale d'une fourche (à quatre branches dans l'exemple). Chaque branche 27 est terminée par une petite barrette 30 de faible largeur, s'étendant transversalement. Ces deux électrodes 23d et 23e sont celles qui se sont révélées parmi les plus efficaces pour le traitement du cuir chevelu une fois qu'une autre électrode 23f est appliquée sur le sujet pour stimuler les follicules pileux.

Selon les autres applications envisagées (traitement cutané ou traitement spécifique du cuir chevelu, notamment) les paramètres de fonctionnement seront adaptés, notamment la valeur de l'amplitude du signal.

L'équipement 111 pour la mise en oeuvre du traitement des rides par pincement tel que représenté figure 4 se compose essentiellement d'un boîtier 112 renfermant un générateur électrique, par exemple un générateur d'impulsions, dont l'intensité peut être réglée au moyen d'un potentiomètre 113 accessible en façade. Une première sortie de ce générateur est reliée à une première électrode 23 montée amovible sur un stylo porte-électrode. Une seconde sortie de ce générateur est reliée à la seconde électrode 24 montée amovible sur un stylo masse porte-électrode.

Le traitement des rides par pincement de la ride entre deux électrodes 23, 24 en appliquant un signal sous une tension variable de 200 à 1200 V, via le transformateur d'impulsions, permet de traiter plus efficacement lesdites rides notamment par une amélioration de la microcirculation artériolo veineuse et lymphatique, une meilleure action sur le métabolisme des couches basales de l'épiderme et une meilleure stimulation des fibroblastes.

## Revendications

1. Appareil de stimulation et de traitement de la peau, comportant des moyens générateurs (11) pour élaborer un signal haute fréquence ayant une fréquence de l'ordre de grandeur de 1 MHz, soit de 0,7MHz à 1,3MHz, modulé en amplitude par un signal basse fréquence ayant une fréquence de l'ordre de grandeur de 50 Hz, soit de 45Hz à 65Hz, sous une tension variable de 200V à 1200V, une première électrode (23), montée sur un stylo porte-électrode de forme cylindrique, reliée à la sortie desdits moyens générateurs (11) et apte à être déplacée sur une première zone de peau à traiter tout en lui appliquant le signal, et une seconde électrode (24), montée sur un stylo masse porte-électrode de forme cylindrique, reliée à la masse et apte à être déplacée sur une zone de peau à traiter adjacente à ladite première zone, la première électrode (23) étant apte à appliquer le signal haute fréquence à la seconde électrode (24) à travers la peau, les deux électrodes (23, 24) comportant chacune une zone d'application allongée continue ou discontinue pour venir pincer la peau, les deux électrodes (23, 24) étant maniables par un utilisateur pour venir se placer de part et d'autre d'une zone de la peau à traiter afin de pincer la peau dans cette zone pour le traitement.

2. Appareil selon la revendication 1, **caractérisé en ce que** lesdits moyens générateurs (11) comprennent un hacheur (12) piloté à ladite haute fréquence et recevant en entrée une tension à basse fréquence.

3. Appareil selon la revendication 2, **caractérisé en ce qu'**il comporte un transformateur haute fréquence (21) ou transformateur d'impulsions qui est connecté entre ledit hacheur (12) et ladite première électrode (23) et qui est adapté à appliquer ledit signal à l'électrode à ladite tension variable de 200V à 1200V.

4. Appareil selon l'une quelconque des revendications précédentes, **caractérisé en ce que** le stylo masse porte-électrode de forme cylindrique comporte une enveloppe métallique, en matériau isolant.

5. Appareil selon l'une quelconque des revendications précédentes, **caractérisé en ce que** la première électrode (23) est amovible par rapport au stylo porte-électrode.

6. Appareil selon l'une quelconque des revendications précédentes, **caractérisé en ce que** la seconde électrode (24) est amovible par rapport au stylo masse porte-électrode.

7. Appareil selon la revendication 5, **caractérisé en ce que** le stylo porte-électrode est associé à un jeu d'électrodes amovibles (23a, 23b, 23c, 23d) de formes différentes, chacune adaptable à un embout de raccordement.

8. Appareil selon la revendication 7, **caractérisé en ce que** la première électrode (23) a la forme d'un peigne, ou d'une fourche ou d'un triangle ou d'une tige à l'extrémité de la laquelle est disposée transversalement une barrette.

9. Appareil selon la revendication 1 à 8, dans laquelle la seconde électrode (24) a une forme triangulaire.

10. Appareil selon l'une quelconque des revendications précédentes, dans lequel la seconde électrode (24) est démontable et remplaçable par une partie métallique (26) apte à être tenue à la main par un sujet pendant un traitement par seulement la première électrode (23) lorsque celle-ci est déplacée sur une zone de peau à traiter tout en lui appliquant le signal.

11. Utilisation d'un appareil selon l'une quelconque des revendications 1 à 10, pour le traitement de la peau entre la première électrode (23) et la seconde électrode (24) en appliquant un signal haute fréquence de l'ordre de grandeur de 1 MHz, soit de 0,7MHz à 1,3MHz, modulé en amplitude par un signal basse fréquence ayant une fréquence de l'ordre de grandeur de 50 Hz, soit de 45Hz à 65Hz, sous une tension variable de 200V à 1200V.

12. Utilisation d'un appareil selon la revendication 11, pour le traitement des rides, cicatrices, dépressions de la peau et cernes par pincement de la peau entre la première électrode (23) et la seconde électrode (24).

13. Utilisation d'un appareil selon la revendication 12, où la première électrode (23) et la seconde électrode (24) sont de forme triangulaire.
